(19) Europäisches Patentamt
European Patent Office
Office européen des brevets

(11) Publication number: **0 297 387 B1**

(12) **EUROPEAN PATENT SPECIFICATION**

(45) Date of publication of patent specification: **05.08.92** (51) Int. Cl.5: **C12Q 1/44**

(21) Application number: **88109819.8**

(22) Date of filing: **21.06.88**

(54) **An improved turbidimetric method for the determination of serum lipase.**

(30) Priority: **01.07.87 US 68975**

(43) Date of publication of application:
**04.01.89 Bulletin 89/01**

(45) Publication of the grant of the patent:
**05.08.92 Bulletin 92/32**

(84) Designated Contracting States:
**AT BE CH DE ES FR GB IT LI NL**

(56) References cited:
**EP-A- 0 014 252**
**EP-A- 0 134 291**
**WO-A-80/02697**
**US-A- 3 436 187**

(73) Proprietor: **ABBOTT LABORATORIES**
**Abbott Park, Illinois 60064(US)**

(72) Inventor: **Robinson, Jack B. Jr.**
**719 Holliday Lane**
**Duncanville, TX 75116(US)**
Inventor: **Levinson, Robert Saul**
**4387 Country Trail**
**Gurnee, IL 60031(US)**
Inventor: **Johnson, Hillard W.**
**4267 Shadowrock Court**
**Gurnee Illinois 60031(US)**
Inventor: **Mowles, Donald L.**
**901 East Golf Road**
**Libertyville Illinois 60048(US)**
Inventor: **Swopes, Herman**
**2015 16th Street**
**North Chicago Illinois 60064(US)**

(74) Representative: **Modiano, Guido et al**
**MODIANO, JOSIF, PISANTY & STAUB Baaderstrasse 3**
**W-8000 München 5(DE)**

EP 0 297 387 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Background of the Invention

The present invention is directed toward an improved method for performing a turbidimetric determination of serum lipase. The method comprises the formation of a microemulsified suspension of lipid particles having an average diameter of less than 0.5 µm. These extremely small lipid particles provide excellent stability and despite their small size an excellent substrate for the lipase-catalyzed hydrolysis to proceed.

The turbidimetric method for determining lipase concentration follows the basic chemical reaction of human pancreatic lipase at a water-lipid interface. In this reaction the human pancreatic lipase catalyzes the hydrolysis of triglycerides with the rapid formation of diglycerides followed by the formation of monoglycerides as follows:

$$\text{lipid} + H_2O \xrightarrow{\text{lipase}} \text{diglyceride} + \text{fatty acid}$$
$$\text{diglyceride} + H_2O \xrightarrow{\text{lipase}} \text{monoglyceride} + \text{fatty acid}$$

The decrease in turbidity of a lipid suspension undergoing lipase-catalyzed hydrolysis is measured by spectrophotometric means. The rate of turbidity decrease is a measure of serum lipase activity.

Lipase reagents for performing a turbidimetric measurement of the lipase activity are well known in the arts. However, difficulty has arisen for formulating a reagent system that has excellent stability and is not dependent upon preparation of the lipid suspension undergoing the lipase catalyzed hydrolysis. Various assays of serum lipase are described in "Assays of Serum Lipase: Analytical and Clinical Considerations", Lott et al, Clin Chem 32/7 1290-1302 (1986).

EP-A-0 014 252 discloses a reagent for determining serum lipase comprising lipid particles having a diameter from 500 to 1000 µm.

### SUMMARY OF THE INVENTION

The present invention is directed toward a turbidimetric method for the determination of serum lipase which comprises adding a serum sample to a suspension of lipid particles having an average diameter of less than 0.5 µm. The change in turbidity of the suspension is then measured to determine the amount of lipase present in the serum sample. A means for reducing the particle size of the lipids to less than 0.5 µm is by microfluidization.

In the method, the lipid particles are present in an amount of from about 0.08 to about 0.3 percent. The preferred lipid particles are triolein.

In another embodiment, the present invention is directed toward a reagent system for determining serum lipase comprising lipid particles having a diameter of less than 0.5 µm. The reagent system has improved stability against sedimentation and coalescence.

In the reagent system, the lipid particles are present in an amount of from about 0.08 to about 0.3 percent and the preferred lipid particles are triolein.

The present invention provides a superior, convenient and accurate assay for serum lipase determination. In particular the reagent system offers stability of upwards to a year or more which is associated the microemulsified lipid dispersion.

### DETAILED DESCRIPTION OF THE INVENTION

Present invention is directed toward an improved turbidimetric method for the determination of serum lipase. The improved method comprises a reagent system having microemulsified lipid particles of less than 0.5 µm in average diameter. The microemulsified lipid particles provide excellent stability and are an excellent substrate for pancreatic lipase and the lipase-catalyzed hydrolysis to proceed.

The lipase reagent system generally comprises a formulation containing lipid particles which are classic substrates for lipase. The term lipid applies to a class of compounds which are soluble in organic solvents and nearly insoluble in water. Chemically, lipids are either compounds that yield fatty acids on hydrolysis or complex alcohols that can combine with fatty acids to form esters and other more complex forms. In the present lipase reagent system lipids can include synthetic or naturally occuring triacylglycerides and other

analogs thereof.

Triolein or glyceryl triolate is a preferred lipid having a molecular formula of $C_{57}H_{104}O_6$,molecular weight 885.4. Triolein is insoluble in water and soluble in solvents such as chloroform, ether, and carbon tetrachloride.

The lipid particles are suspended in the reagent system by various physical means which can include vigorous magnetic stirring or manual stirring. The quantity of lipid particles employed is generally from about 0.08 percent to about 0.3 percent (percent is based on grams per 100 ml), more preferably the lipid particles are present in an amount from about 0.10 percent to about 0.15 percent. In addition to the lipid particles the serum lipase reagent system generally comprises a buffer to adjust the pH of the reagent system, an antimicrobial, a bile acid, a calcium containing activator to increase the rate of catalysis, and stabilizers.

The quantity of the various components can be varied to prepare a serum lipase reagent system depending on the instrumentation employed to perform the serum lipase assay. The reagent system is formed into a suspension and is microemulsified or microfluidized by passage through a microfluidizer such that the lipid particles have an average diameter of less than 0.5 µm. At this dimension the reagent system is extremely stable against sedimentation and coalescence.

Microfluidization is a technique developed to prepare extremely fine liquid-liquid and liquid-solid dispersions. This technique is based on a submerged jet principle in which fluidized streams interact at ultrahigh velocity in precisely defined microchannels. Microfluidizers are commercially available through Microfluidics Corporation, Newton, Massachusetts.

After the lipid particles are microfluidized to form an emulsion, additional solid components of the reagent system can be conveniently added. Finally, the reagent system is adjusted to a predetermined pH to prepare it for use in an assay for serum lipase.

The components which are employed to formulate a reagent system for assays of serum lipase are described below. While the individual components of the reagent system are important to the instant invention it is the microemulsification of the lipid particles which is understood to provide the unique advantages to a reagent system employed for serum lipase and forms the subject matter of this invention.

The lipid particles are employed as a substrate for the pancreatic enzymes present in the serum lipase. The lipid particles are processed to provide sufficient substrate for the hydrolysis of triglycerides to be observed and yet to provide a low enough scattering of visible light so as to be measurable in various photometric systems. The useful concentration of the lipid microfluidized particles would be in the range of from about 0.08 percent to about 0.3 percent based upon grams per 100 ml. A preferred lipid particle is triolein having a final assay concentration of approximately 0.1125 percent.

A useful component in the reagent system is sodium azide, which is employed as an antimicrobial. A useful range for the antimicrobial component is from about 0.1 to about 0.5 percent, more preferably 0.25 percent. Sodium deoxycholate is another component commonly employed in a lipase reagent system to inhibit other not specific hydrolytic enzymes. Useful concentrations of this compound are from about 10 to about 25 millimolar (mM), more preferably 13.5 mM. In the absence of a colipase, the sodium deoxycholate would also inhibit the lipase of interest, so this assay's class would be called a "colipase rescue" type and is well known in lipase assays. In addition to sodium deoxycholate, other biosol derivatives at varying concentration can be employed.

Tricine, N-Tris(hydroxymethyl)methylglycine, is also employed in a reagent system and serves a dual role as a buffer to prevent pH change during reaction and as a calcium binder to prevent deleterious effects due to a precipitation phenomena of various calcium salts. A useful range for tricine would be from about 10 to about 100 mM depending on other assay components, more preferably 33.75 mM.

Another component which is useful in lipase reagent systems is Triton X-100 a registered trademark for a mixture of compounds commercially available from Rohm and Hass, Philadelphia, PA) which is employed for two reasons. One, it helps to prevent an increase in absorbance due to serum addition and, two, it serves to stabilize the microemulsion. Lipase assays could be made without it but matrix effects would be present. While Triton X-100 is a preferred stabilizer or surfactant, there are many other non-ionic detergents which can be used. A useful range for the non-ionic detergent, or preferably Triton X-100 is in a range of from about 0 to about 0.4 percent, more preferably 0.165 percent.

Colipase is present in a lipase reagent system as a necessary activator of lipase and can be present in an amount from about 3 to about 20 mg per liter. Generally, however, economic considerations due to the high cost of colipase prevent the higher concentrations from being used. A more preferred concentration of colipase is approximately 6.25 mg per liter. Another necessary component of a lipase reagent system is calcium chloride which is present as an activator. Useful concentrations of calcium chloride are from about 0.1 to about 10 mM, depending on other assay components.

Dextran sulfate can also be present in the lipase reagent system to prevent serum effects and is classically used as a serum clarifier. It may also serve as a calcium binder. A useful amount is from about 0.03 to about 0.15 percent, more preferably 0.06 percent.

The pH of the lipase reagent system is generally adjusted from 7 to 9, more preferably 8.6. The pH can be adjusted by any of a number of means of acid or base combinations which do not interfere with the other reagent components.

All of the above listed components are useful in formulating a lipase reagent system. The formulation is generally determined to provide an efficient, economical and reliable serum lipase assay with no specific effects due to serum and having good long term stability. In view of the large number of reagent components which can be employed to prepare a serum lipase reagent system, the various combinations cannot be described here. The critical aspect of the present invention, however, is that the lipid particle size is microfluidized into an emulsion, microemulsion, having an average diameter of less than 0.5 µm, which assists in providing an effective, economical and reliable lipase assay reagent. More importantly, the very small lipid particles provide a suspension which is very stable against sedimentation and coalescence. This means that the particular reagent system can be transported and stored for long periods of time avoiding any necessity to provide dry components to a clinical test site which must then be reconstituted prior to performing the assay. Thus the present reagent system need not be formulated prior to use which increases the accuracy of the assay by providing a consistent reagent assay system which is not dependent upon the reconstitution procedure or reconstitution solution employed. For instance, one need not be concerned about the various qualities of water that would be employed to reconstitute a dry reagent system.

<u>EXAMPLE 1</u> - Physical Comparison of Fresh and 30 Day Old Microemulsified Lipase Reagent

In this experiment, a base emulsion was diluted with calcium activator solution at day one and stored on-board the ABBOTT SPECTRUM® clinical laboratory analyzer (Abbott Laboratories, North Chicago, Illinois) for 30 days with septum cap (8 degrees Celsius, with exposure to air). At day 30 this solution was pipetted manually, along with a freshly prepared solution, into a cuvette, and the monochromatic apparent absorbances of the emulsion solutions recorded. The results of these measurements are shown in Table I.

The base emulsion consisted of 0.1125% triolein, microemulsified 20 days before the start of the experiment at (14000 psi) 965,3 bar and 3 passes through the microfluidizer, 6.25 mg/liter colipase, 13.5 mM sodium deoxycholate, 33.7 mM tricine buffer, pH 8.6 and supplemented later with standard antimicrobial, antioxidant and activating agents, as described above. Final calcium concentration was 1.1 mM.

Table I

| Wavelength(nm) | Fresh | 30 Days Old |
|---|---|---|
| 340 | 1.6101 | 1.6211 |
| 364 | 1.5012 | 1.5101 |
| 380 | 1.4027 | 1.4079 |
| 404 | 1.3020 | 1.3088 |
| 412 | 1.2688 | 1.2749 |
| 452 | 1.0718 | 1.0757 |
| 484 | 0.9690 | 0.9714 |
| 500 | 0.9342 | 0.9364 |

It is concluded that since particle size is a component of the factors which cause a variance in apparent absorbance with wavelength in light scattering experiments, that the particle size of the microemulsified reagent has not significantly changed in the 30 days storage and that no sedimentation or coalesence has occurred. In view of this stability it can be reasonably assumed that even in the absence of destabilizing agents the subject microemulsified lipase reagent would be stable for at least one year. Storage of the base emulsion was at 4 degrees Celsius, in a tightly capped bottle and with no added calcium.

<u>EXAMPLE 2</u> - Enzymatic Activity of Microemulsified Lipase Reagent

The values shown in Table II, below, represent the change in apparent absorbance at 340 nanometers of the lipase reagent system described in Example 1 upon addition of the indicated amount of human

serum, to which purified human lipase had been added to represent a pathological sample of interest in a clinical laboratory. This would represent approximately 600 Rick units of lipase activity, a middle range pathological sample. The normal usage for values as these would be expressed as an average change in absorbance over the range represented, i.e., -0.007 ± 0.001 and -0.008 ± 0.001 change in apparent absorbance per minute for the 2 reagents caused by the 2 µl (microliter) serum sample added to 236 µl of lipase reagent in the cuvette of an ABBOTT SPECTRUM® clinical laboratory analyzer. Similarly, for the 5 µl samples the averages would be -.018 to 0.023 ± 0.005. The use of negative rates is a convention indicating a decrease in apparent absorbance.

Table II

| Time(min) | Enzyme amount | Fresh | 30 days old |
|---|---|---|---|
| 4-5 | 2 µl | -0.006 | -0.007 |
| 5-6 | 2 µl | -0.008 | -0.008 |
| 6-7 | 2 µl | -0.007 | -0.010 |
| 7-8 | 2 µl | -0.007 | -0.008 |
| 4-5 | 5 µl | -0.018 | -0.026 |
| 5-6 | 5 µl | -0.019 | -0.027 |
| 6-7 | 5 µl | -0.017 | -0.023 |
| 7-8 | 5 µl | -0.018 | -0.017 |

The conclusions drawn from the data are that the reagent system is responsive in a linear fashion to added lipase activity, and that there is not a significant difference in response to added enzyme between freshly prepared reagent and reagent aged 30 days, and that the response of the change in apparent absorbance is proportional to the amount of serum added.

EXAMPLE 3 - Description of Control Experiments

The reagent system described in Example 1 does not show any change in apparent absorbance upon addition of serum or serum to which purified lipase has been added without the addition of the calcium chloride solution. Additionally, heating the serum or serum to which purified lipase has been added to 56 degrees Celsius for 2 hours, and then adding it to the lipase reagent gives no change in apparent absorbance. Both of these experiments are indicative of properties known to be associated with pancreatic lipase present in human serum and are indicative that the reagent system so described is specific for this enzyme activity.

## Claims

**1.** A turbidimetric method for the determination of serum lipase comprising:
adding a serum sample to a suspension of lipid particles having an average diameter of less than 0.5 µm; and
measuring a change in turbidity of said suspension to determine the amount of lipase present in said serum sample.

**2.** The method of Claim 1 wherein said lipid particles are present in an amount of from about 0.08 to about 0.3 percent.

**3.** The method of Claim 1 wherein said lipid particles are triolein.

**4.** A reagent system for determining serum lipase comprising:
lipid particles having an average diameter of less than 0.5 µm.

**5.** The reagent system of Claim 4 wherein said lipid particles are present in an amount of from about 0.08 to about 0.3 percent.

**6.** The reagent system of Claim 4 wherein said lipid particles are triolein.

## Revendications

1. Procédé turbidimétrique pour déterminer la lipase sérique comprenant:
   l'addition d'un échantillon de sérum à une suspension de particules lipidiques ayant un diamètre moyen inférieur à 0,5 µm; et
   la mesure d'une variation de la turbidité de ladite suspension pour déterminer la quantité de lipase présente dans ledit échantillon de sérum.

2. Le procédé de la revendication 1 dans lequel lesdites particules lipidiques sont présentes en une quantité d'environ 0,08 à environ 0,3 pour cent.

3. Le procédé de la revendication 1 dans lequel lesdites particules lipidiques sont de la trioléine.

4. Système réactif pour déterminer la lipase sérique comprenant:
   des particules lipidiques ayant un diamètre moyen inférieur à 0,5 µm.

5. Le système réactif de la revendication 4 dans lequel lesdites particules lipidiques sont présentes en une quantité d'environ 0,08 à environ 0,3 pour cent.

6. Le système réactif de la revendication 4 dans lequel lesdites particules lipidiques sont de la trioléine.

## Patentansprüche

1. Turbidimetrisches Verfahren zur Bestimmung von Serum-Lipasen, welches Verfahren die folgenden Stufen umfaßt:
   - Hinzufügen einer Serumprobe zu einer Suspension von Lipidteilchen mit einem durchschnittlichen Durchmesser von weniger als 0,5 µm; und
   - Messen einer Veränderung in der Trübung dieser Suspension, um die Menge der Lipasen zu bestimmen, welche in dieser Serumprobe vorliegen.

2. Verfahren nach Anspruch 1, wobei die Lipidteilchen in einer Menge von etwa 0,08 bis etwa 0,3 % vorliegen.

3. Verfahren nach Anspruch 1, wobei die Lipidteilchen Triolein sind.

4. Reagenziensystem zur Bestimmung von Serum-Lipasen, welches umfaßt:
   - Lipidteilchen mit einem durchschnittlichen Durchmesser von weniger als 0,5 µm.

5. Reagenziensystem nach Anspruch 4, wobei die Lipidteilchen in einer Menge von etwa 0,08 bis etwa 0,3 % vorliegen.

6. Reagenziensystem nach Anspruch 4, wobei die Lipidteilchen Triolein sind.